# EUROPEAN PATENT SPECIFICATION

(11) **EP 1 541 116 B1**
(45) Date of publication and mention of the grant of the patent: **12.04.2006**
(21) Application number: 03027984.8
(22) Date of filing: 05.12.2003
(51) Int. Cl.: A61K 8/04, A61K 8/81, A61K 8/98, A61Q 5/06

(54) **Hair styling composition**
Haarstyling Zusammensetzung
Composition de coiffure

(43) Date of publication of application: 15.06.2005
(73) Proprietor: KPSS-Kao Professional Salon Services GmbH, 64297 Darmstadt (DE)
(72) Inventor: Bräutigam, Ina, 64297 Darmstadt (DE); Hermes, Frank, 64342 Seeheim-Jugenheim (DE)

(56) References cited:
- EP-A- 0 315 541
- EP-A- 0 756 859
- WO-A-03/070208
- DE-A- 2 630 560
- FR-A- 2 411 001
- US-A- 4 268 500
- 'Hair vital styling mousse', [Online] Retrieved from the Internet: <URL:www.good-products.de> [retrieved on 2004-04-15]
- 'Yogurtene' TRADE MARKS JOURNAL no. 6491, 01 August 2003,
- DWECK A.: 'Column for Soap, Perfumery and Cosmetics', [Online] January 2003, Retrieved from the Internet: <URL:www.dweckdata.com> [retrieved on 2004-05-10]

## Description

This invention relates to a hair styling composition comprising spray dried yoghurt powder. Compositions can as well be used suitably for hair caring purposes.

Compositions have been known for conditioning and/or styling hair for some time. They customarily comprise in an aqueous and/or aqueous-alcoholic medium hair conditioning substances such as cationic polymers, cationic surfactants and as well hair styling polymers of anionic, cationic, non-ionic and/or amphoteric or zwitterionic character.

Although state of the art is developed to a certain extent there is still need for improvements in hair styling area.

Natural ingredients have always been attractive to the consumer. Compositions containing natural ingredients especially derived from the edible ones have always been regarded as safer than any other composition mainly based on synthetic raw materials. Designing modern cosmetic formulations solely on the basis of natural ingredients will probably take more time in the future.

For example in EP 756 859, a styling composition is disclosed comprising grafted silicone polymer and two additional polymers of different ionic characters selected from group of anionic, cationic, non-ionic or amphoteric ones. In the whole length of the patent specifications nothing is said on the use of natural ingredients and especially yoghurt powder.

In a US patent US 4,268,500, use of yoghurt as a natural product for treating scalp and hair is disclosed. Natural yoghurt is used simply by rubbing onto hair and scalp according to the method disclosed therein. The document does not disclose any use of yoghurt or its extracts and its powders in any modern cosmetic formulation such as hair styling compositions.

In recently published PCT application, WO 03/070208, use of yogurt is disclosed for improvement of non-oxidative hair colouring. The disclosure contains as well shampoo and conditioner compositions to be used for improving colour retention on non-oxidatively coloured hair. The document, however, does not deal with hair styling compositions for hair.

The subject of the present invention is providing an aqueous and/or aqueous-alcoholic hair-styling composition comprising at least one styling polymer and spray dried yoghurt powder. The compositions of the present invention can be formulated as an aerosol hair spray and as well as solutions to be sprayed onto hair using a mechanical pump device or applied as a solution/lotion. Gel type compositions are as well in the scope of the present invention.

Upon application of the compositions of present invention, hair setting can be improved. Properties of hair such as manageability, shine, volume and body and elasticity are improved too.

For a successful hair styling process, sufficient amount of composition of the present invention must be applied onto a freshly washed and/or shampooed hair.

Yoghurt powder is a raw material prepared by spray drying of natural yoghurt after completion of fermentation. Yoghurt powder comprises the following major components:
- approximately 53.5% lactose,
- approximately 25% proteins,
- approximately 7.5% lactic acid,
- approximately 5% minerals and trace elements,
- approximately 1 % vitamines, and
- approximately 2% lipids.

Compositions of the present invention comprise yoghurt powder in a concentration range of 0.01 to 10%, preferably 0.01 to 5%, more preferably 0.01 to 3 by weight calculated to total composition.

The styling polymer or polymers is/are selected from the anionic, non-ionic, cationic and/or amphoteric or zwitterionic ones. The styling composition of the present invention comprises at least one styling polymer. Certainly, the similar effects are achieved when more than one polymer of the same type or as well of different types in combination are used.

Non-ionic polymers are selected from the ones soluble in water and/or alcohol and/or in alcohol water mixtures, at any ratio. Under the definition of soluble in alcohol and alcohol water mixture, it should be understood that the polymer is soluble in lower alcohols such as ethanol, n-propanol or isopropanol and in their mixtures with water, at any ratio

Suitable non-ionic polymer is first of all vinylpyrrolidon polymers either homopolymers or copolymers with, especially, vinylacetate. Those are known with the trade name "Luviskol" as homopolymers Luviskol K 30, K 60 or K 90 as well copolymers Luviskol VA 55, VA 64 from BASF AG.

Further non-ionic polymer suitable for compositions of the present invention is vinylpyrrolidone/vinylacetae/vinylpropionate copolymer known with the trade name Luviskol VAP 343 as well from BASF.

Natural non-ionic polymers are as well suitable for the composition of the present invention. Those are such as cellulose, chitosan, guar gum, neutralised shellac and their derivatives.

Cationic polymers are of greater variability in their structure found to be suitable for the styling composition of the present invention. It has been found out that especially those of cationic cellulose type polymers known as Polymer JR type from Amerchol such as Polyquaternium 10 or cationic guar gum known with trade name Jaguar from Rhône-Poulenc and chemically for example guar hydroxypropyl trimonium chloride, are preferred ones.

Furthermore, it has especially been found suitable those cationic polymers known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28. Among those the most preferred one is the Polyquaternium 11 as well known with its trade name Gafquat from ISP and as Luviquat PQ from BASF.

As well those polymers known with their CTFA category name Quaternium are suitable. Those are for example Quaternium-8, Quaternium-14, Quaternium-15, Quatemium-18, Quatemium-22, Quaternium-24, Quaternium-26, Quaternium-27, Quatemium-30, Quaternium-33, Quatarnium-37, Quaternium-53, Quaternium-60, Quaternium-61, Quatemium-72, Quaternium-78, Quaternium-80, Quaternium-81, Quatemium-81, Quatemium-82, Quatemium-83 and Quatemium-84.

In this context, reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The cationic polymers also include the quatemized products of graft polymers from organopolysiloxanes and polyethyl oxazolines described in EP-A 524 612 and EP-A 640 643.

Amphoteric or zwitterionic polymers, preferably be used in mixture with at least one non-ionic and/or cationic polymers, are also found to be suitable for styling composition of the present invention. Examples to the preferred ones are copolimerisate of n-octylacrylamide, acrylic or metahcrylic acid and tert.-butylaminoethylmethacrylate known with its trade name Amphomer, copolymer of methacryloylethylbetaine and alkyl methacrylate known as Yukaformer, terpolymer of metahcrylic or acrylic acid and itaconoic acid and a basic monomer of mono or dialkylaminoalkyl acrylate or methacrylate or acryla of methacrylamide known with the trade name Aquaflex SF 40.

As amphoteric polymers which can be used alone or in mixture with at least one additional cationic and/or nonionic polymer, special reference is here made in particular to copolymers of N-octyl acrylamide, (meth)acrylic acid and tert.-butylaminoethyl methacrylate of the type "Amphomer®"; copolymers from methacryloyl ethyl betaine and alkyl methacrylates of the type "Yukaformer®", e.g., the butyl methacrylate copolymer "Yukaformer® Am75"; copolymers from monomers containing carboxyl groups and sulfonic groups, e.g., (meth)acrylic acid and itaconic acid, with monomers such as mono- or dialkyl aminoalkyl (meth)acrylates or mono- or dialkyl aminoalkyl (meth)acrylamides containing basic groups, in particular amino groups; copolymers from N-octyl acrylamide, methyl methacrylate, hydroxypropylmethacrylate, N-tert.-butyl aminoethyl methacrylate and acrylic acid, as well as the copolymers known from US-A 3,927,199.

Anionic polymers are as well suitable for styling compositions of the present invention. Suitable ones are vinyl alkyl ether, in particular methyl vinyl ether/maleic acid copolymers, obtained by hydrolysis of vinyl ether/maleic anhydride copolymers, distributed under the trade name "Gantrez® AN or ES". These polymers may also be partly esterified, as for example, "Gantrez® ES 225" or "ES 435", the ethyl ester of an ethyl vinyl ether/maleic acid copolymer, or the butyl or isobutyl ester thereof.

Further useful anionic polymers are in particular vinyl acetate/crotonic acid or vinyl acetate/vinyl neodecanoate/crotonic acid copolymers of the type "Resyn®"; sodium acrylate/vinyl alcohol copolymers of the type "Hydagen® F", sodium polystyrene sulfonate, e.g., "Flexan® 130"; ethyl acrylate/acrylic acid/N-tert.-butyl acrylamide copolymers of the type "Ultrahold®"; vinyl pyrrolidone/vinyl acetate/itaconic acid copolymers, acrylic acid/acrylamide copolymers or the sodium salts thereof of the type "Reten®"; etc.

Concentration of styling polymers of anionic, cationic, non-ionic and/or amphoteric or zwitterionic character is in the range of 0.05 - 10%, preferably 0.05 - 7.5% and most preferably 0.05 - 5% by weight, calculated to the total composition.
The styling composition of present invention can comprise hair-conditioning agents. Conditioning agents can be selected from oily substances, non-ionic substances, cationic amphiphilic ingredients, cationic polymers (as mentioned above) or their mixtures. Oily substances are selected from such as silicone oils, either volatile or non-volatile, natural and synthetic oils. Among silicone oils those can be added to the pre-treatment composition include dimethicone, dimethiconol, polydimethylsiloxane, DC fluid ranges from Dow Corning, natural oils such as olive oil, almond oil, avocado oil, weizenkeim oil, ricinus oil and the synthetic oils, such as mineral oil.

Non-ionic conditioning agents can as well be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula I or II, respectively,

R₁CO(OCH₂CH₂)ₙOH formula I

R₁CO(OCH₂CH₂)ₙOOCR₂ formula II

where R₁ and R₂ are independent from each other saturated, unsaturated or branched or non-branched alkyl chain with 7 to 21 C atoms and n is typically 2 - 100.

Hair styling composition can contain further cationic amphiphilic conditioning ingredients according to the formula III. 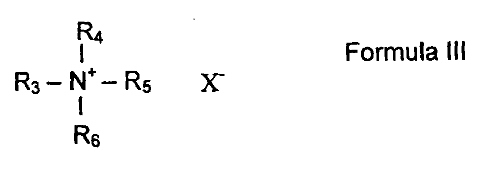 where R₃ is a saturated or unsaturated, branched or non-branched alkyl chain with 8-22 C atoms or

R₇CONH(CH₂)ₙ

where R₇ is saturated or unsaturated, branched or non-branched alkyl chain with 7-21 C atoms and n has typical value of 1 - 4 or

R₈COO(CH₂)ₙ

where R₈ is saturated or unsaturated, branched or non-branched alkyl chain with 7-21 C atoms and n has typical value of 1 - 4, and
R₄ is H or unsaturated or saturated, branched or non-branched alkyl chain with 1 - 22 C atoms or

R₇ CO NH (CH₂)ₙ

or

R₈COO(CH₂)ₙ

where R₇, R₈ and n are same as above.

R₅ and R₆ are H or lower alkyl chain with 1 to 4 Carbon atoms, and X is typically chloride, bromide or methosulfate.

Typical examples of those ingredients are cetyl trimethly ammonium chloride, stear trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate. Those can at the same time serve as solubilizing agents for those ingredients difficult to integrate into the formulations.

The cationic polymers mentioned above used for achieving styling are as well found to be suitable for hair conditioning purposes when formulated into the compositions of the present invention.

Typical concentration range for any of the conditioners mentioned above can be 0.01 - 5% by weight, preferably 0.01 - 3.5% by weight and more preferably 0.05 - 2.5% by weight calculated to the total composition.

The compositions according to the invention comprise at least one surfactant, whereby the total amount of surfactants can preferably range from about 0.1 % to 5%, in particular about 0.2% to 2.5% by weight, calculated to the total composition.

It is basically possible to use all known anionic, nonionic, amphoteric or zwitterionic and/or cationic surfactants, of which nonionic and/or amphoteric or zwitterionic surfactants are especially preferred.

Suitable nonionic surfactants are, e.g., the various sorbitan esters, such as polyethylene glycol sorbitan stearic acid ester, fatty acid polyglycol esters or mixed condensates of ethyleneoxide and propyleneoxide, as they are on the market, for example, under the trade name "Pluronics".

Additionally useful nonionic surfactants are amineoxides. Such amineoxides are state of the art, for example C₁₂-C₁₈-alkyl dimethyl amineoxides such as lauryl dimethyl amineoxide, C₁₂-C₁₈-alkyl amidopropyl or -ethyl amineoxides, C₁₂-C₁₈-alkyl di(hydroxyethyl) or (hydroxypropyl) amineoxides, or also amineoxides with ethyleneoxide and/or propyleneoxide groups in the alkyl chain. They are on the market, for example, under the trade names "Ammonyx® ", "Aromox® " or "Genaminox® ".

Further nonionic surfactant components are, for example, fatty acid mono- and dialkanol-amides, such as coco fatty acid monoethanolamide and myristic fatty acid monoisopro-panolamide, which can also be used as additional foam enhancers, as well as long-chain alkyl polyglucosides.

Further nonionic components in the composition according to the invention are C₁₀₋C₂₂-fatty alcohol ethoxylates.

Especially suited C₁₀-C₂₂-fatty alcohol ethers are those known by the common names "Laureth", "Myristeth", "Oleth", "Ceteth", Deceth", "Steareth" and "Ceteareth", e.g. the alkyl polyglycol ethers named according to the CTFA nomenclature by adding the number of the ethyleneoxide molecules, for example "Laureth-16". The average ethoxylation degree ranges from about 5 to about 25, preferably from about 10 and about 20.

Useful amphoteric or zwitterionic surfactants are in particular the various known betaines such as fatty acid amido alkyl betaines and sulfobetaines, for example, lauryl hydroxy sulfobetaines, long-chain alkylamino acids such as cocoaminoacetate, cocoaminopropionate and sodium cocoamphopropionate and -acetate.

In detail, it is possible to use betaines of the structure 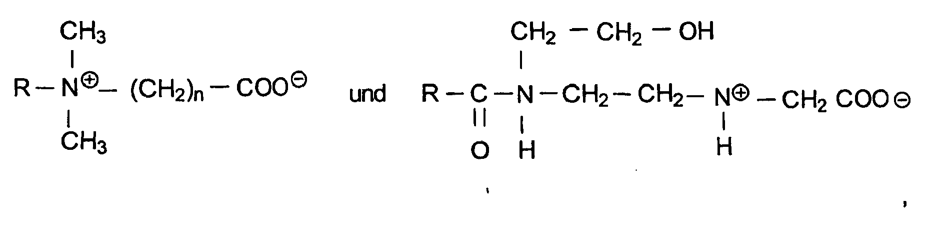 wherein R is a C₈-C₁₈-alkyl group and n is 1 to 3,
sulfobetaines of the structure 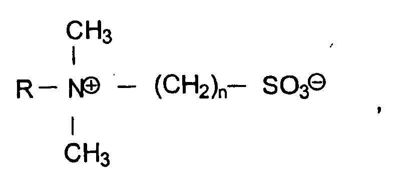 wherein R is a C₈-C₁₈-alkyl group and n is 1 to 3,
and amidoalkyl betaines of the structure 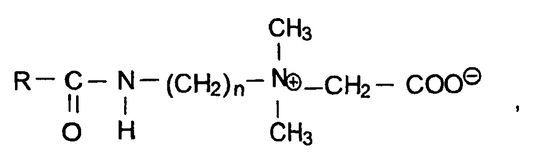 wherein R is a C₈-C₁₈-alkyl group and n is 1 to 3.

Preferred are fatty acid amidoalkyl betaines, especially cocoamidopropyl betaine, and cocoamphoacetate and -propionate, in particular the sodium salts thereof.

Especially preferred are mixtures of cocoamidopropyl betaine and cocoamphoacetate, in particular in a weight proportion of 3:1 to 1:3, especially 2:1 to 1:1.

Possible anionic surfactants are, for example, C₈-C₂₀-alkyl polyether carboxylic acids or the salts thereof of the formula

R-(A)ₙ-O-CH₂COOX,

wherein R is a C₈-C₂₀-alkyl or alkenyl group, preferably a C₁₂-C₁₄-alkyl group, n is a number from 1 to 20, preferably 2 to 17, A is an ethylene- and/or propylene oxide group, and X is H or preferably a cation of the group sodium, potassium, magnesium, and ammonium, which can, optionally, be hydroxyalkyl substituted. Such products have been known and on the market for some time, for example, under the trade names "AKYPO® " and "AKYPO-SOFT® ".

Further suitable anionic surfactants are, for example, those of the sulfate, sulfonate, carboxylate and alkyl phosphate type, especially, of course, those customarily used in hair treatment compositions, in particular the known C₁₀-C₁₈-alkyl sulfates, and especially the respective ether sulfates, for example, C₁₂-C₁₄-alkyl ether sulfate, lauryl ether sulfate, especially with 1 to 4 ethylene oxide groups in the molecule, furthermore monoglyceride(ether) sulfates, fatty acid amide sulfates, obtained by ethoxylation and subsequent sulfatation of fatty acid alkanolamides, and the alkali salts thereof, acyl isethionates as well as the salts of long-chain mono- and dialkyl phosphates, which are mild, skin-compatible detergents.

A further preferred anionic surfactant group which can be incorporated in the compositions according to the invention are C₈-C₂₂-acyl aminocarboxylic acids or the water-soluble salts thereof, for example, N-lauroyl glutamate, in particular as sodium salt. Further suitable N-acyl aminocarboxylic acids are N-lauroyl sarcosinate, N-C₁₂₋C₁₈-acyl asparagic acid, N-myristoyl sarcosinate, N-oleoyl sarcosinate, N-lauroyl methyl alanine, N-lauroyl lysine and N-lauroyl aminopropyl glycine, preferably in the form of the water-soluble alkali salts or ammonium salts, in particular the sodium salts thereof.

It is also possible to incorporate mixtures of several anionic surfactants, for example, a mixture of an α-olefine sulfonate and a sulfosuccinate, preferably in a ratio of 1 : 3 to 3 : 1, or of an ether sulfate and an alkyl amidoether carboxylic acid.

Solubilizers of anionic, nonionic, cationic and amphoteric character can as well be used in combination in the conditioners of the present invention. In this case, the total concentration of the solubilizer in the final product should, as a rule, not exceed 2%, prefereably 1.5% and more preferably 1.0% by weight calculated to the total composition. It should be noted that the solubilizers preferred are nonionic and cationic surfactants.

Any of the cationic surfactant mentioned above can serve as well as solubilizer. For solubilizing purpose the preferred ones are those with single alkyl chain such as cetrimonium chlorise, steratrimonium chloride.

The non-ionic surfactants are found to be particularly suitable as solubilizer. Those ethoxylates of hydrogenated castor oil such as PEG 40 hydrogenated castor oil are found to be particularly suitable know with the trade name Cremophor from BASF.

Other suitable non-ionic and anionic and amphoteric or zwitterionic surfactants can be found in the monograph of Schrader, K. H., Grundlagen und Rezepturen der Kosmetika, pages 681 - 695.

The concentration of solubilizers in the formulation should not exceed 2%, preferably 1.5% most preferably 1% by weight, calculated to the total composition.

The compositions according to the invention can comprise propellant and/or mixtures of propellants, preferably in an amount up to 50% by weight, calculated to the total aerosol foam composition, in the case that an aerosol preparation is produced.

Useful propellants are, for example, hydrocarbons such as propane, n-butane, i-butane and the mixtures thereof, dimethyl ether, and/or carbon dioxide. The pressure in the aerosol container preferably ranges from about 4 to 6 bar.

The cooling agents are selected from the well known ones menthol or mentyl lactate or their mixtures. Certainly, the used solvents such as alcohol have also been proven to show cooling effect on skin. According to the present invention it has been found out that in the presence of one or more above-mentioned cooling agents, perceivable effect is understood to be much better and/or improved. The total concentration of the cooling agents, menthol or menthyl lactate or their mixtures, is in the range of 0.05 to 1.5%, preferably 0.05 to 1%, more preferably 0.05 to 0.75% by weight calculated to the total weight of the composition.

Compositions of the present invention can comprise UV filters either for stabilization of the product colour or for protection of hair from environmental influences such as loss of elasticity, loss of hair colour (bleaching effect of sun light). The UV-absorbing substance is preferably selected from the following compounds:
4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxybenzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzophenone or the sodium salt thereof, 2-hydroxy-4-octyloxybenzophenone, 2-hydroxy-4-methoxy-4'-methylbenzophenone, 3-benzylidenecampher, 3-(4'-sulfo)-benzylidenebornane-2-one and the salts thereof and/or 3-(4'-methyl benzylidene)-DL-campher.

The preferred amount of the UV-absorber ranges from about 0.01 % to 2.5%, preferably from 0.05 % to 1 % by weight, calculated to the total composition.

The compositions of the present invention can comprise hair-restructuring agents. The hair restructuring agents preferred are especially the ones disclosed in the German patent DE 197 51 550 C2.

One of the known hair restructuring agents is ceramide type of compound with the general formula 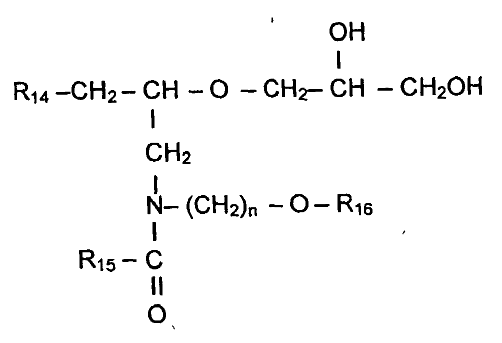 where R¹⁴ and R¹⁵ are independent from each other alkyl- or, alkenyl group mit 10 to 22 carbon atoms, R¹⁶ is methyl, ethyl, n-propyl or isopropyl group and n is a number between 1 to 6, preferably 2 or 3.

Other preferred hair restructuring agents are fatty acids with 10 to 24 carbon atoms and especially with 16 to 24 carbon atoms.

Sterols, especially the phytosterols, are as well preferred hair restructuring agents as disclosed in the above mentioned German patent. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

The concentration of ceramide in the compositions of the present invention can be in the range of 0.01 to 2 and especially 0.01 to 1% by weight calculated to the total weight of the composition. The fatty acids may be contained at a level of 0.01 to 2.5% and epecially 0.01 to 1% by weight calculated to the total weight of the composition. Phytosterol concentration of the conditioners is less than 1% and preferably less than 0.5% by weight calculated to the total weight of the composition. It should be noted without limiting the use of those ingredients the effect of those hair restructuring ingredients is especially elevated when used in combination with penetration enhancers.

Moreover, the hair styling compositions according to the invention can comprise all compounds customarily found in hair styling and/or conditioning compositions, such as perfumes, dyestuffs both for coloration of the products, as well as direct-acting dyestuffs for the coloration of human hair in accordingly increased concentrations, solvents and solubilizers, pH-regulants, preservatives, vitamins, plant extracts, neutralizing agents for the incorporated polymers, etc., in suitable, known amounts.

The pH-value of the compositions according to the invention is not critical and can basically vary between about 2 and 8.5, in particular about 3 and 8.

The following examples are to illustrate the invention, but not limiting it.

### Example 1

### Pumpspray

| | % |
|---|---|
| Ethanol | 90.0 |
| Polyquaternium-11 | 0.2 |
| Polyvinylcaprolactam | 1.0 |
| Yoghurt powder | 0.3 |
| Menthol | 0.3 |
| Fragrance | 0.2 |
| PEG-40 Hydrogenated Castor oil | 0.2 |
| Water | add 100.0 |

Polymers are dissolved in ethanol, menthol, and fragrance is dissolved/mixed first with PEG-40 hydrogenated castor oil and than added to the solution of polymers in ethanol. Finally, the composition is made up to 100% by adding water.

### Example 2

### Aerosolspray

| | |
|---|---|
| Ethanol | 35.0 |
| PVP (Luviskol K 30) | 0.2 |
| Vinylacetate/crotonic acid copolymer | 3.6 (Aristoflex A 60) |
| Yoghurt powder | 0.2 |
| Fragrance | 0.2 |
| Water | 34.1 |
| Dimethylether | 38.0 |
| n-pentane | 12.0 |
| Neutralizing agent | q.s. pH 8.0 |

The composition is prepared in the same way as in Example 1 except that the propellant, dimethylether (DME), is added after transferring the solution into its final vessel.

### Example 3

### Pumpspray

| | |
|---|---|
| PVP (Luviskol K 90) | 0.3 |
| Acrylates/Octylacrylamide Copolymer | 1.0 |
| Aminomethyl Propanol | 0.25 |
| Fragrance | 0.2 |
| Yoghurt powder | 0.3 |
| Ethanol | add 100 |

The composition is prepared in the same way as in Example 1.

### Example 4

### Pumpspray

| | % |
|---|---|
| Polyquaternium-11 | 0.25 |
| VP/VA Copolymer ((Luviskol VA 64W) | 2.0 |
| Yoghurt powder | 0.5 |
| Menthyl lactate | 0.2 |
| Ethanol | 40 |
| Fragrance | 0.2 |
| PEG-40 Hydrogenated Castor oil | 0.2 |
| Water | add 100 |

The composition is prepared in the same way as in Example 1.

### Example 5

### Pumpspray

| | |
|---|---|
| | % |
| Shellac (neutralized) | 1.0 |
| PVP | 0.2 |
| Menthol | 0.3 |
| Yoghurt powder | 0.1 |
| Octylmethoxycinnamate | 0.3 |
| Fragrance | 0.2 |
| Water | 10.0 |
| Ethanol | add 100 |

The composition is prepared in the same way as in Example 1.

### Example 6

### Pumpspray

| | % |
|---|---|
| Chitosan | 0.1 |
| VPNA Copolymer (Luviskol VA 64W) | 2.0 |
| Yogurt powder | 0.5 |
| Fragrance | 0.2 |
| PEG-40 Hydrogenated castor oil | 0.2 |
| Ethanol | 10 |
| Water | add 100 % |

The composition is prepared in the same way as in Example 1.

### Example 7

### Styling gel

| | % |
|---|---|
| PVP (Luviskol K 90) | 4.0 |
| VP/VA Copolymer (Luviskol VA 64W) | 4.0 |
| Panthenol | 0.5 |
| Yoghurt powder | 1.5 |
| PEG-40 Hydrogenated castor oil | 0.2 |
| Glycerol | 10.0 |
| Ethanol | 10.0 |
| Fragrance, preservative | q.s. |
| Neutralizing agents | q.s to pH 6.0 |
| Water | add 100 |

The composition is prepared in the same way as in Example 1.

### Example 8

### Styling gel

| | % |
|---|---|
| Acrylates/Octylacrylamide copolymer | 2.0 (Amphomer HC) |
| VP/VA Copolymer (Luviskol VA 64W) | 8.0 |
| Acrylates / C10-30 cetyl acrylate | |
| Crospolymer | 0.6 |
| Panthenol | 0.5 |
| Yoghurt powder | 0.8 |
| PEG-40 Hydrogenated castor oil | 0.3 |
| Propylene glycol | 5.0 |
| Ethanol | 10.0 |
| Fragrance, preservative | q.s. |
| Neutralizing agents | q.s to pH 6.5 |
| Water | ad 100 |

The composition is prepared in the same way as in Example 1.

### Example 9

### Styling mousse

| | % |
|---|---|
| Polyquaternium-11 | 2.0 (Gafquat 440) |
| VPNA Copolymer (Luviskol VA 64W) | 4.0 |
| Cetrimonium chloride | 0.4 |
| Laureth-23 | 0.2 |
| Yoghurt powder | 0.8 |
| PEG-40 Hydrogenated castor oil | 0.2 |
| Ethanol | 5.0 |
| Fragrance, preservative | q.s. |
| Water | ad 100 |

The composition is prepared in the same way as in Example 1. The solution is filled into an aerosol can with a propellant mixture of propane/butane at a liquid composition to propellant ratio of 90:10.

### Example 10

### Styling mousse

| | % |
|---|---|
| Polyquaternium-46 | 4.0 (Luviquat Hold) |
| VP/VA Copolymer (Luviskol VA 64W) | 8.0 |
| Dicocoylethyl hydroxyethylmonium | |
| methosulfate | 0.6 |
| Laureth-23 | 0.3 |
| Yoghurt powder | 0.8 |
| PEG-40 Hydrogenated castor oil | 0.2 |
| Benzophenone-3 | 0.3 |
| Ethanol | 5.0 |
| Fragrance, preservative | q.s. |
| Water | ad 100 |

The composition is prepared in the same way as in Example 1. The solution is filled into an aerosol can with a propellant mixture of propane/butane at a liquid composition to propellant ratio of 95:5.

### Example 11

### Pump spray for damaged hair

| | |
|---|---|
| PVP (Luviskol K 90) | 0.3 |
| Acrylates/Octylacrylamide Copolymer | 1.0 |
| Aminomethyl Propanol | 0.25 |
| Ceramide compund according to formula (R¹=C₁₅H₃₁; R²=C₁₆H₃₃; R³=H; R⁴=H; n=2) | 0.05 |
| Behenic acid | 0.1 |
| Yoghurt powder | 0.2 |
| Avocadin | 0.05 |
| Fragrance | 0.2 |
| PEG-40 Hydrogenated castor oil | 0.2 |
| Ethanol | add 100 |

## Claims

1. R Aqueous and/or aqueous-alcoholic hair styling composition **characterised in that** it comprises at least one hair styling polymer and spray dried yoghurt powder.

2. Hair styling composition according to claim 1 **characterised in that** styling polymer is selected from non-ionic, anionic, cationic and/or amphoteric or zwitterionic ones.

3. Hair styling composition according to claims 1 and 2 **characterised in that** it comprises a second polymer as well selected from non-ionic, anionic, cationic and/or amphoteric or zwitterionic ones.

4. Hair styling composition according to any of the preceding claims **characterised in that** it comprises hair styling polymers at a concentration of 0.05 - 10% by weight, calculated to the total composition.

5. Hair styling composition according to any of the preceding claims **characterised in that** it comprises spray dried yoghurt powder at a concentration of 0.01-10% by weight, calculated to the total composition.

6. Hair styling composition according to any of the preceding claims **characterised in that** it comprises hair conditioning agents at a concentration of 0.05 - 5% by weight calculated to the total composition.

7. Hair styling composition according to any of the preceding claims **characterised in that** it comprises anionic, non-ionic, cationic and/or amphoteric or zwitterionic surfactants.

8. Hair styling composition according to any of the preceding claims **characterised in that** it comprises dyestuffs both for coloration of the products, as well as direct-acting dyestuffs for the coloration of human hair, solubilizers, pH-regulants, preservatives, vitamins, plant extracts, neutralizing agents for the incorporated polymers.

9. Method for application of hair styling composition according to claims 1 - 3 **characterised in that** composition is applied to hair in form of a pump spray from a bottle equipped with a mechanical pump or in the form of an aerosol spray or in the form of a aerosol foam or as a solution/lotion.

10. Method according to claim 9 **characterised in that** in the case that the composition is applied to hair in the form of an aerosol hair spray or an aerosol foam then it contains at least one propellant gas.

## Patentansprüche

1. Wässrige und/oder wässrig-alkoholische Haarstyling-Zusammensetzung, **dadurch gekennzeichnet, dass** sie mindestens ein Haarstyling-Polymer und ein sprühgetrocknetes Yoghurt-Pulver enthält.

2. Haarstyling-Zusammensetzung nach Anspruch 1, **dadurch gekennzeichnet, dass** das Styling-Polymer aus nichtionischen, anionischen, kationischen und/oder amphoterischen oder zwitterionischen Polymeren ausgewählt ist.

3. Haarstyling-Zusammensetzung nach Anspruch 1 und 2, **dadurch gekennzeichnet, dass** sie ein zweites Polymer, ausgewählt aus nichtionischen, anionischen, kationischen und/oder amphoterischen oder zwitterionischen, enthält.

4. Haarstyling-Zusammensetzung nach einem der Ansprüche 1 bis 3, **dadurch gekennzeichnet, dass** sie Styling-Polymere in einer Menge von 0,05 bis 10 Gew.-%, berechnet auf die Gesamtzusammensetzung, enthält.

5. Haarstyling-Zusammensetzung nach einem der Ansprüche 1 bis 4, **dadurch gekennzeichnet, dass** sie sprühgetrocknetes Yoghurt-Pulver in einer Menge von 0,01 bis 10 Gew.-%, berechnet auf die Gesamtzusammensetzung, enthält.

6. Haarstyling-Zusammensetzung nach einem der Ansprüche 1 bis 5, **dadurch gekennzeichnet, dass** sie Haarpflegesubstanzen in einer Menge von 0,05 bis 5 Gew.-%, berechnet auf die Gesamtzusammensetzung, enthält.

7. Haarstyling-Zusammensetzung nach einem der Ansprüche 1 bis 6, **dadurch gekennzeichnet, dass** sie anionische, nichtionische, kationische und/oder amphoterische oder zwitterionische Tenside enthält.

8. Haarstyling-Zusammensetzung nach einem der Ansprüche 1 bis 7, **dadurch gekennzeichnet, dass** sie sowohl Farbstoffe für die Produktanfärbung, als auch direktziehende Farbstoffe zur Färbung menschlicher Haare, Lösungsvermittler, pH-Regulatoren, Konservierungsmittel, Vitamine, Pflanzenextrakte und Neutralisationsmittel für die eingearbeiteten Polymere enthält.

9. Verfahren zur Verwendung einer Haarstyling-Zusammensetzung nach den Ansprüchen 1 bis 3, **dadurch gekennzeichnet, dass** die Zusammensetzung in Form eines Pumpsprays aus einer Flasche, die mit einer mechanischen Pumpe ausgerüstet ist, in Form eines Aerosol-Sprays, eines Aerosol-Schaums oder als Lösung oder Lotion auf das Haar aufgetragen wird.

10. Verfahren nach Anspruch 9, **dadurch gekennzeichnet, dass** beim Auftragen der Zusammensetzung auf das Haar, in der Form eines Haarsprays oder eines Aerosol-Schaums, dieses mindestens ein Treibmittel enthält.

## Revendications

1. Composition de coiffure aqueuse et/ou aqueuse-alcoolique, **caractérisée en ce qu'**elle comprend au moins un polymère de coiffure et une poudre de yaourt séchée par atomisation.

2. Composition de coiffure selon la revendication 1, **caractérisée en ce que** le polymère de coiffure est choisi parmi les polymères non ioniques, anioniques, cationiques et/ou amphotères ou zwittérioniques.

3. Composition de coiffure selon les revendications 1 et 2, **caractérisée en ce qu'**elle comprend un deuxième polymère également choisi parmi les polymères non ioniques, anioniques, cationiques et/ou amphotères ou zwittérioniques.

4. Composition de coiffure selon l'une quelconque des revendications précédentes, **caractérisée en ce qu'**elle comprend des polymères de coiffure en une concentration de 0,05 à 10 % en poids, rapportée à la composition totale.

5. Composition de coiffure selon l'une quelconque des revendications précédentes, **caractérisée en ce qu'**elle comprend une poudre de yaourt séchée par atomisation en une concentration de 0,01 à 10 % en poids, rapportée à la composition totale.

6. Composition de coiffure selon l'une quelconque des revendications précédentes, **caractérisée en ce qu'**elle comprend des agents de conditionnement des cheveux en une concentration de 0,05 à 5 % en poids, rapportée à la composition totale.

7. Composition de coiffure selon l'une quelconque des revendications précédentes, **caractérisée en ce qu'**elle comprend des tensioactifs anioniques, non ioniques, cationiques et/ou amphotères ou zwittérioniques.

8. Composition de coiffure selon l'une quelconque des revendications précédentes, **caractérisée en ce qu'**elle comprend des colorants à la fois pour la coloration des produits, ainsi que des colorants à action directe pour la coloration de cheveux humains, des solubilisants, des régulateurs de pH, des conservateurs, des vitamines, des extraits végétaux, des agents de neutralisation pour les polymères incorporés.

9. Procédé d'application de la composition de coiffure selon les revendications 1 à 3, **caractérisé en ce que** la composition est appliquée sur les cheveux sous forme d'une pulvérisation à pompe à partir d'une bouteille équipée d'une pompe mécanique ou sous forme d'une pulvérisation en aérosol ou sous la forme d'une mousse en aérosol ou sous forme d'une solution/lotion.

10. Procédé selon la revendication 9, **caractérisé en ce que**, dans le cas où la composition est appliquée sur les cheveux sous forme d'une pulvérisation capillaire en aérosol ou d'une mousse en aérosol, elle contient alors au moins un gaz propulseur.
